Europäisches Patentamt

European Patent Office

Office européen des brevets

(19)

(11) Numéro de publication : **0 633 461 A2**

(12) **DEMANDE DE BREVET EUROPEEN**

(21) Numéro de dépôt : **94400908.3**

(22) Date de dépôt : **27.04.94**

(51) Int. Cl.⁶ : **G01N 1/38**, G01N 33/00, G01N 1/24, F26B 21/08

(30) Priorité : **21.05.93 FR 9306113**

(43) Date de publication de la demande :
**11.01.95 Bulletin 95/02**

(84) Etats contractants désignés :
**BE DE DK ES FR GB GR IE IT LU NL PT SE**

(71) Demandeur : **SOCIETE D'AUTOMATISME DE PRODUCTION**
**Parc d'Activité de l'Alambic,**
**Merpins**
**F-16100 Cognac (FR)**

(72) Inventeur : **Aime, Bruno**
**13, rue des Charmilles**
**F-86340 Les Roches Premaries (FR)**

(74) Mandataire : **Rinuy, Santarelli**
**14, avenue de la Grande Armée**
**F-75017 Paris (FR)**

(54) **Procédé et dispositif pour mesurer l'humidité d'un air chaud et installation de séchage à air chaud comportant ce dispositif.**

(57)    L'invention propose de diluer dans des conditions connues l'air chaud, par exemple celui qui circule dans une étuve de séchage (1), avec de l'air frais, par exemple de l'air extérieur, dont l'humidité est connue, afin d'obtenir un air mélangé dont les caractéristiques conviennent aux capteurs d'humidité conventionnels, par exemple un psychromètre ; et de déduire l'humidité de l'air chaud à partir des conditions connues de dilution, de l'humidité connue de l'air frais et de l'humidité mesurée de l'air mélangé.

Pour diluer l'air chaud, on utilise de préférence un mélangeur (14) comportant une enceinte (15) connectée à une source de succion (16).

Fig.1

EP 0 633 461 A2

L'invention a trait sous son aspect le plus général à la mesure de l'humidité de l'air, et sous un aspect plus particulier aux installations de séchage à air chaud.

Pour effectuer une telle mesure avec une bonne précision, on connaît déjà divers procédés et capteurs qui sont utilisables tant que la température de l'air reste inférieure à 100°, et s'il existe des capteurs à semi-conducteurs qui sont utilisables à des températures supérieures, ils ne sont pas adaptés à des ambiances du genre de celles qu'on rencontre dans les installations de séchage à air chaud, qui sont proches de la saturation (4 à 10 kg de vapeur d'eau par kg d'air sec à 120-140°), et parfois chargées en poussières.

L'invention vise à permettre de mesurer l'humidité d'un tel air chaud dans des conditions satisfaisantes.

L'invention propose à cet effet, sous un premier aspect, un procédé pour mesurer l'humidité d'un air chaud, caractérisé en ce qu'on dilue dans des conditions connues ledit air chaud avec de l'air frais dont l'humidité est connue afin d'obtenir un air mélangé dont la température est plus basse, on mesure l'humidité dudit air mélangé, et on déduit l'humidité de l'air chaud à partir des conditions connues de dilution, de l'humidité connue de l'air frais et de l'humidité mesurée de l'air mélangé.

Grâce à l'invention, en choisissant les conditions de dilution pour que la température de l'air mélangé soit suffisamment basse pour permettre de mesurer son humidité avec une méthode conventionnelle ayant une bonne précision et un temps de réponse correct, on rend finalement possible de déduire l'humidité de l'air chaud avec des performances satisfaisantes.

Selon des caractéristiques préférées pour obtenir ledit air mélangé, on utilise une source de succion, une chambre de mesure qui ne s'ouvre que sur la source de succion et sur une chambre de mélange, et ladite chambre de mélange qui ne s'ouvre que sur la chambre de mesure, sur l'air chaud et sur l'air frais, la dilution s'effectuant en régime continu dans la chambre de mélange tandis que la chambre de mesure est traversée par un flux régulier d'air mélangé qui provient de la chambre de mélange et se dirige vers la source de succion.

L'air mélangé est ainsi automatiquement obtenu de façon simple et commode.

Selon d'autres caractéristiques préférées, pour connaître les conditions de dilution :
- on prédétermine le calibrage des orifices à travers lesquels la chambre de mélange s'ouvre respectivement sur l'air chaud et sur l'air frais ;
- on maintient à une valeur constante prédéterminée la pression de la source de succion ; et
- on mesure ou on estime la pression et la température de l'air frais et de l'air chaud en amont de leur orifice calibré respectif.

On a ainsi uniquement des opérations simples à effectuer au cours du temps, le maintien de la pression de la source de succion à une valeur constante pouvant se faire automatiquement grâce à un régulateur de pression, les mesures de température et de pression susvisées ne posant quant à elles pas de difficultés particulières.

Selon d'autres caractéristiques préférées pour éviter des phénomènes de condensation, on réchauffe ledit air chaud en amont de la chambre de mélange, et on chauffe les parois des chambres de mélange et de mesure.

Il est en effet important de lutter contre tous les phénomènes de condensation, puisque si ceux-ci se produisaient, une certaine quantité de vapeur d'eau ne ferait plus partie de l'air, et la mesure serait faussée.

Les caractéristiques précitées permettent de lutter de façon simple et efficace contre les différentes causes possibles de condensation, notamment la baisse de pression et de température de l'air chaud dans son trajet jusqu'à la chambre de mélange, le fait d'avoir les parois des chambres de mélange et de mesure qui seraient plus froides que l'air contenu dans celles-ci, et le fait que la température à l'intérieur de ces chambres ne serait pas suffisamment élevée pour que toute l'eau contenue dans l'air puisse se maintenir en phase vapeur.

Selon d'autres caractéristiques préférées pour mesurer l'humidité de l'air mélangé, on utilise un psychromètre disposé dans ladite chambre de mesure.

L'invention est en effet compatible avec les conditions d'utilisation relativement délicates de cet appareil (voir ci-après), le choix de celui-ci permettant de bénéficier de l'excellente précision qu'il peut offrir.

L'invention propose sous un deuxième aspect un dispositif pour mesurer l'humidité d'un air chaud, convenant à la mise en oeuvre du procédé qui vient d'être exposé, caractérisé en ce qu'il comporte :
- un mélangeur pour diluer dans des conditions connues ledit air chaud avec de l'air frais dont l'humidité est connue, afin d'obtenir un air mélangé dont la température est plus basse ; et
- un capteur pour mesurer l'humidité dudit air mélangé, destiné à être relié à un calculateur adapté à déduire l'humidité de l'air chaud à partir des informations fournies par ledit capteur, de l'humidité connue de l'air frais, et des conditions connues de dilution.

Selon des caractéristiques préférées :
- ledit mélangeur comporte une enceinte destinée à communiquer avec la source d'air chaud, avec la source d'air frais et avec une source de succion ; ladite enceinte étant divisée en une chambre de mélange et une chambre de mesure ; ladite chambre de mélange ne communiquant qu'avec la source d'air chaud, la source d'air frais et la chambre de mesure ; ladite chambre de mesure ne communiquant qu'avec la chambre de mélange et la source de succion de sorte que la dilution s'effectue en régime continu dans la chambre de mélange tandis que la chambre de mesure est traversée par un flux régulier d'air mélangé qui provient de la chambre de mélange et se dirige vers la source de succion ; ledit capteur pour mesurer l'humidité de l'air mélangé étant disposé dans ladite chambre de mesure ;

- ledit mélangeur comporte un premier orifice à calibrage prédétérminé entre ladite chambre de mélange et la source d'air chaud, et un deuxième orifice à calibrage prédéterminé entre la chambre de mélange et la source d'air frais ;
- le dispositif comporte en outre :
  . des capteurs pour mesurer la pression et la température de l'air chaud en amont dudit premier orifice à calibrage prédéterminé, et
  . des capteurs pour mesurer l'humidité, la température et la pression de l'air frais en amont dudit deuxième orifice à calibrage prédéterminé,

la pression de la source de succion (16) étant prévue pour rester constante ;
- ledit mélangeur comporte des moyens pour réchauffer ledit air chaud entre la source d'air chaud et l'amont dudit premier orifice à calibre prédéterminé ;
- ledit mélangeur comporte des moyens pour chauffer les parois des chambres de mélange et de mesure.

Selon d'autres caractéristiques préférées, ledit capteur pour mesurer l'humidité de l'air mélangé est un psychromètre, et ledit mélangeur comporte dans ladite chambre de mesure un conduit de section prédéterminée dans lequel est disposé le psychromètre, ledit conduit communiquant avec ladite chambre de mélange à travers un troisième orifice à calibrage prédéterminé.

Le fait d'obliger l'air à passer par cet orifice, qui est de section beaucoup plus petite que celle de la chambre de mélange, améliore la qualité du mélange de l'air chaud et de l'air frais.

Cet orifice et ce conduit permettent en outre de fixer la vitesse de l'air dans lequel est placé le psychromètre, le conduit protégeant également le psychromètre du rayonnement émis par les parois de l'enceinte si elles sont plus chaudes que l'air mélangé dans lequel baigne le psychromètre.

Selon d'autres caractéristiques préférées, le psychromètre comporte un tube d'alimentation en eau qui a dans la chambre de mesure une longueur adaptée à amener à la température de la chambre de mesure l'eau qui se dépose sur la sonde de température humide.

On réchauffe ainsi de manière particulièrement simple et fiable l'eau de mouillage de la sonde de température humide.

Selon d'autres caractéristiques préférées, ledit mélangeur comporte un filtre entre la chambre de mélange et la chambre de mesure.

Ceci permet d'éviter d'encrasser le capteur d'humidité et notamment, s'il s'agit d'un psychromètre, de souiller l'habillage destiné à être imbibé d'eau de la sonde de température humide.

Sous un troisième aspect, l'invention vise une installation de séchage à air chaud, caractérisée en ce qu'elle comporte un dispositif tel que précédemment exposé pour mesurer l'humidité de l'air chaud de séchage.

Une telle installation, dans laquelle on connaît donc avec une bonne précision l'humidité de l'air chaud de séchage, offre l'avantage de permettre de piloter finement les caractéristiques de celui-ci eu égard à l'état du chargement à sécher.

Selon des caractéristiques préférées, l'installation comporte des moyens pour que ledit dispositif puisse mesurer l'humidité de l'air chaud de séchage respectivement en amont et en aval du chargement à sécher.

En comparant les mesures amont et aval, on peut connaître avec une bonne précision quelle est l'humidité que cède le chargement à l'air de séchage, et donc savoir dans quel état se trouve ce chargement.

Selon d'autres caractéristiques préférées, qui offrent l'avantage de permettre d'automatiser plus ou moins l'installation de séchage :
- l'installation comporte un automate qui pilote au moins un de ses organes en fonction des informations d'humidité issues dudit dispositif pour mesurer l'humidité de l'air chaud de séchage, et ;
- ledit automate pilote en fonction des informations fournies par le dispositif de mesure d'humidité au moins l'un des organes suivants :
  - organe d'humidification de l'air de séchage ;
  - organe d'introduction d'air neuf ;
  - organe d'extraction d'air de séchage ;
  - organe de circulation d'air ;
  - organe de chauffage.

L'exposé de l'invention sera maintenant poursuivi par la description d'un exemple de réalisation, donnée ci-après à titre illustratif et non limitatif, en référence aux dessins annexés. Sur ceux-ci :
- la figure 1 montre de façon schématique une installation de séchage à air chaud conforme à l'invention ;
- la figure 2 montre une portion d'une variante de cette installation où est prévue une deuxième prise d'air chaud de séchage ;
- la figure 3 est une vue schématique en élévation-coupe plus détaillée du dispositif pour mesurer l'humidité de l'air chaud ;

- la figure 4 est un agrandissement de la partie centrale de ce dispositif ;
- la figure 5 est une élévation-coupe en vue de dessus de cette portion ; et
- la figure 6 est une vue partielle similaire à la figure 3 montrant une variante pour la disposition de la prise d'air frais.

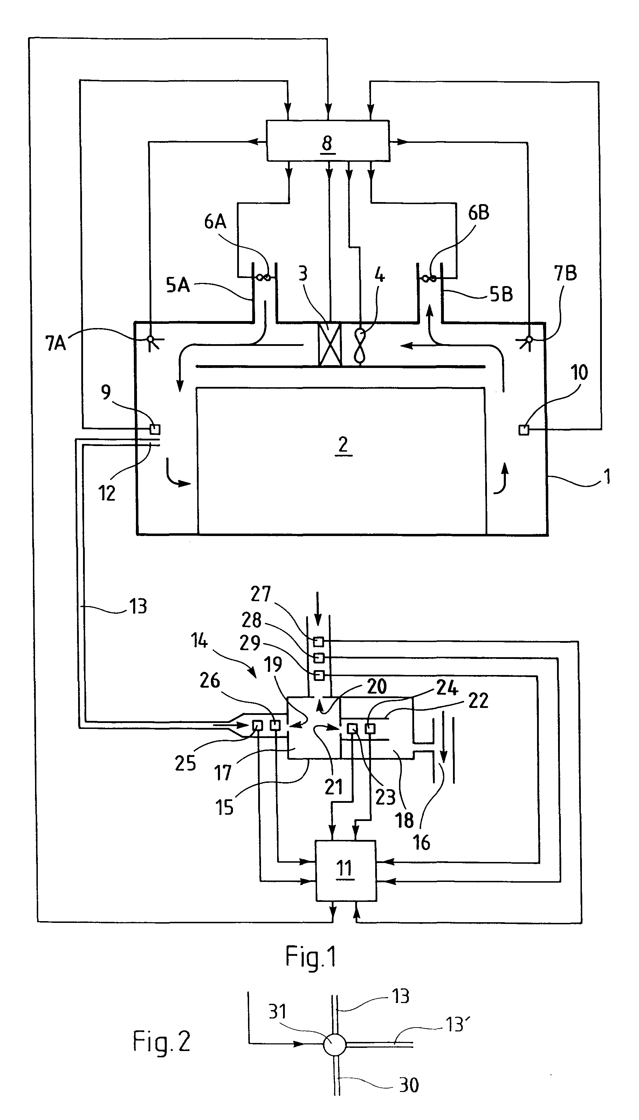

L'installation de séchage montrée sur la figure 1 comporte une étuve 1 dans laquelle est disposé un chargement 2 de produit à sécher, ici du bois massif. L'étuve 1 comporte un brûleur ou une batterie de chauffage 3, un ventilateur 4 de mise en circulation de l'air dans l'étuve, deux gaines 5A et 5B d'extraction ou d'introduction d'air dans chacune desquelles est prévu un volet ou ventilateur 6A ou 6B, respectivement, et des dispositifs d'humidification de l'air 7A ou 7B.

Pour faire circuler l'air dans l'étuve 1 comme indiqué par les flèches, le ventilateur 4 tourne dans le sens où l'air qu'il expulse traverse le brûleur ou la batterie de chauffage 3, de l'air neuf est introduit grâce à la gaine 5A et au volet ou au ventilateur 6A, de l'humidité est éventuellement rajoutée à l'air grâce au dispositif 7A, l'air chaud et éventuellement humidifié vient ensuite au contact du chargement 2 qu'il contourne et/ou traverse puis il est aspiré par le ventilateur 4, la gaine 5B servant à extraire une partie de l'air chaud, dans une proportion fixée grâce au volet ou ventilateur 6B.

L'étuve 1 est prévue pour que le sens de circulation de l'air puisse être inversé, le ventilateur 4 aspirant alors l'air provenant du brûleur ou de la batterie de chauffage 3, la gaine 5B servant à l'introduction d'air neuf, le dispositif 7B à son humidification, et la gaine 5A à l'extraction de l'air.

L'installation montrée sur la figure 1 comporte pour son pilotage un automate 8 qui est rélié, afin de commander leur fonctionnement, au brûleur ou à la batterie de chauffage 3, au ventilateur 4, aux volets ou ventilateurs 6A ou 6B et aux humidificateurs 7A et 7B.

L'automate 8 est également relié, pour recevoir les informations à partir desquelles il va piloter les organes précités, à des sondes de température 9 et 10 placées de part et d'autre (en amont et en aval) du chargement 2, ainsi qu'à un calculateur 11 qui lui fournit un signal représentatif de l'humidité de l'air prélevé dans l'étuve 1 par la prise 12, qui se trouve en amont du chargement 2 lorsque l'air de séchage circule comme illustré.

La prise d'air 12 communique avec une canalisation calorifugée 13 qui communique elle-même avec un mélangeur 14 prévu pour diluer dans des conditions connues l'air chaud de séchage avec de l'air frais, ici l'air extérieur, dont l'humidité est connue, ceci afin d'obtenir un air mélangé dont les caractéristiques sont compatibles avec les conditions d'utilisation d'un capteur conventionnel prévu pour mesurer l'humidité de cet air mélangé. La température de celui-ci est par exemple de l'ordre de 80°.

Le mélangeur 14 comporte une enceinte 15 communiquant, en outre de la source d'air chaud constituée par l'étuve 1 et de la source d'air frais formée par l'air extérieur, avec une source de succion 16. Cette enceinte 15 est divisée en une chambre de mélange 17 et en une chambre de mesure 18, la chambre 17 ne communiquant qu'avec la source d'air chaud, la source d'air frais et la chambre de mesure 18, celle-ci ne communiquant qu'avec la chambre de mélange 17 et la source de succion 16.

La source de succion extrait de l'air de la chambre 18 qui aspire à son tour de l'air dans la chambre de mélange 17 qui elle-même aspire de l'air dans l'étuve 1 et à l'extérieur. Il s'effectue ainsi dans la chambre 17 un mélange en régime continu entre l'air de l'étuve 1 et l'air extérieur, et la chambre 18 est traversée par un flux régulier d'air mélangé qui provient de la chambre 17 et est aspiré par la source de succion 16.

Les trois orifices 19, 20 et 21 de la chambre 17, par lesquels celle-ci communique respectivement avec la source d'air chaud (l'étuve 1), la source d'air frais (l'extérieur) et la chambre de mesure 18 sont tous à calibrage prédéterminé ; et dans la chambre 18 il est prévu un conduit 22 à section prédéterminée qui communique avec la chambre 17 à travers l'orifice 21.

Le calibrage des orifices 19 et 20 permet de fixer les conditions de dilution de l'air chaud par l'air frais tandis que le calibrage de l'orifice 21 et la section du conduit 22 permettent de fixer la vitesse de l'air mélangé dans ce conduit.

Dans ce dernier est disposé un capteur pour mesurer l'humidité de l'air mélangé, ici un psychromètre formé par un ensemble à deux sondes de température, respectivement une sonde 23 de température sèche et une sonde 24 de température humide revêtue d'un habillage imbibé d'eau portée à la température de l'air mélangé.

Pour le bon fonctionnement du psychromètre, on dimensionne le calibrage de l'orifice 21 et la section du conduit 22 pour que l'air mélangé ait une vitesse de l'ordre de 2 m/s au niveau des sondes 23 et 24 avec un écoulement en régime établi.

Pour connaître les conditions de dilution de l'air chaud par l'air frais, il faut connaître non seulement le calibrage des orifices 19 et 20 mais aussi des données de température et de pression de sorte que l'on a prévu juste en amont de l'orifice 19 un capteur de température 25 et un capteur de pression 26, tandis qu'en amont de l'orifice 20 on a prévu un capteur de pression 27 et un psychromètre avec une sonde de température sèche 28 et une sonde de température humide 29.

Les capteurs et sondes 23 à 29 sont chacun relié au calculateur 11 qui est adapté à déduire l'humidité de l'air chaud à partir des informations qu'ils fournissent, et à délivrer à l'automate 8 un signal correspondant à l'humidité déduite.

On peut en effet obtenir l'humidité de l'air chaud à partir de l'humidité de l'air mélangé, de l'humidité de l'air frais et des conditions de dilution avec la formule suivante :

$$X_{ip} = \frac{AX_i(\delta + X_{id}) + \delta(X_i - X_{id})}{A(\delta + X_{id}) - (X_i - X_{id})}$$

dans laquelle $X_{ip}$ est l'humidité absolue de l'air chaud, $X_i$ est l'humidité absolue de l'air mélangé, $X_{id}$ est l'humidité absolue de l'air frais, A est un facteur représentatif des conditions de dilution, et $\delta$ est une constante égale à 0,62198 représentative du rapport des masses molaires de la vapeur d'eau par rapport à l'air sec.

$X_i$ et $X_{id}$ sont déduits à partir des informations fournies par les sondes 23, 24 et 28, 29 respectivement, conformément à la méthode psychrométrique.

Le facteur A représentatif des conditions de dilution est obtenu par la formule suivante :

$$A = K\frac{p_2 - P_3}{P_{ATM} - P_3}\frac{T_{ATM}}{T_2}\frac{P_2}{P_{ATM}}$$

où K est une constante qui dépend du calibrage des orifices 19 et 20 et qui est connue par étalonnage, $P_2$ est la pression de l'air chaud fournie par le capteur 26, $P_3$ est la pression dans la chambre 17 qui est constante et connue par étalonnage (la pression de la source de succion 16 est maintenue constante à cet effet grâce à un régulateur de pression), $P_{ATM}$ est la pression de l'air frais fournie par le capteur 27, $T_{ATM}$ est la température de l'air frais fournie par la sonde de température sèche 28, et $T_2$ est la température de l'air chaud fournie par le capteur 25.

On notera qu'on peut considérer en pratique que la pression de la source de succion reste constante tant qu'elle est située dans une certaine plage relativement étendue, de sorte qu'on peut directement utiliser une source d'air comprimé conventionnelle malgré le caractère grossier de sa régulation de pression.

Dans la variante montrée sur la figure 2, ce n'est plus directement la conduite 13 qui arrive au mélangeur 14, mais une conduite 30 qui est reliée à la conduite 13 et à la conduite 13' par une vanne trois voies 31 qui met la conduite 30 en communication soit avec la conduite 13 soit avec la conduite 13', cette dernière communiquant avec une prise d'air située dans l'étuve 1 du côté de la charge 2 qui opposé à la prise 12, la vanne 31 étant reliée à l'automate 8 qui la pilote. Lorsque l'air de séchage circule comme indiqué par les flèches, et que la vanne 31 met en communication les conduites 30 et 13, alors la source d'air chaud avec laquelle communique le mélangeur 14 est l'air de séchage en amont du chargement 2, tandis que c'est l'air de séchage en aval lorsque la vanne 31 met la conduite 30 en communication avec la conduite 13'.

On notera que le dispositif formé par le mélangeur 14 et les capteurs et sondes reliés au calculateur 11 a un temps de réponse relativement rapide et en tout cas considérablement plus rapide que les capteurs à semi-conducteurs précités dont le temps de réponse est d'environ 10 mn. On peut donc dans certaines conditions considérer que l'humidité de l'air de séchage amont et aval n'a pas varié pendant que l'on déduisait respectivement l'humidité en amont et l'humidité en aval, et en définitive considérer que la différence entre les deux valeurs d'humidité déduites correspond à l'humidité cédée à l'air de séchage par le chargement 2.

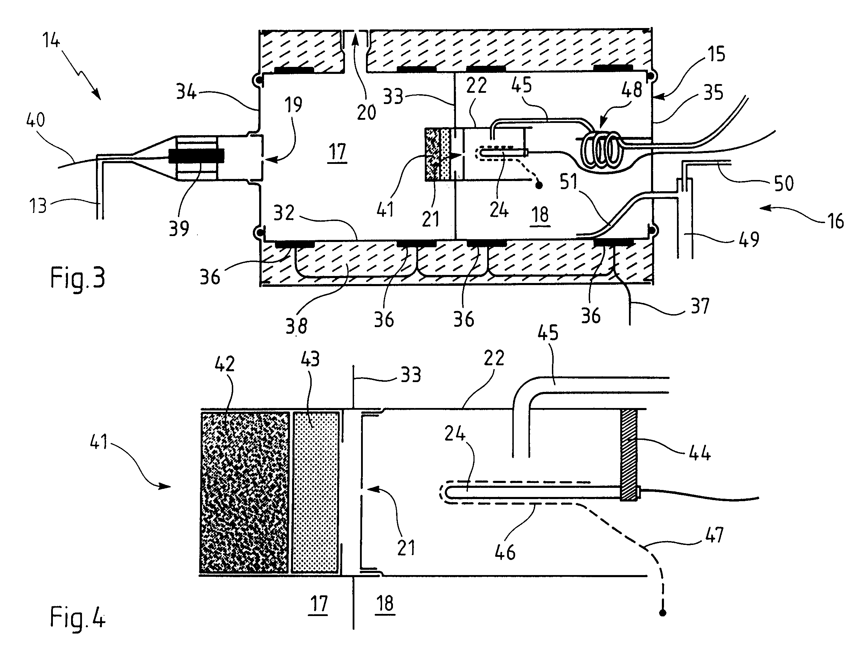

Le dispositif pour mesurer l'humidité de l'air chaud est montré plus en détail sur les figures 3 à 5, les capteurs et sondes 25 à 29 n'étant toutefois pas représentés.

L'enceinte 15 a une forme générale cylindrique dont la paroi latérale extérieure 32 est commune aux chambres 17 et 18, celles-ci étant séparées par une paroi annulaire transversale 33, chacune des chambres 17 et 18 étant fermées à l'opposé de la paroi 33 par une paroi transversale 34 ou 35, respectivement.

Des colliers chauffants électriques 36, alimentés par le câble 37, sont prévus du côté externe de la paroi 32, et comme celle-ci ainsi que les parois 33 et 34 en contact avec l'extérieur sont métalliques, la chaleur communiquée par les colliers 36 se transmet dans toutes ces parois. Pour éviter des déperdition de chaleur, le mélangeur 14 comporte une couche 38 d'isolant thermique autour de la paroi 32. On peut également en prévoir du côté externe des parois 34 et 35.

Grâce aux colliers 36, on chauffe les parois des chambres 17 et 18 en contact avec l'extérieur à une température plus élevée que celle de l'air contenu dans ces chambres, afin d'éviter les phénomènes de condensation tant sur leurs parois qu'à l'intérieur même de ces chambres, la chaleur apportée permettant à l'air qui s'y trouve de prendre une température telle que toute la vapeur d'eau qu'il contient reste en phase vapeur (température telle que la pression de vapeur saturante est supérieure à celle du point de rosée). On a prévu de répartir plusieurs colliers chauffants 36 le long de la paroi 32 afin la température soit le plus uniforme possible dans l'enceinte 15 en vue d'éviter la condensation dûe à des changements de température de l'air.

La paroi 33 et le tube 22 sont normalement à la même température que l'air contenu dans l'enceinte 15 puisqu'ils sont entièrement plongés dans cet air. Le tube 22 peut ainsi servir à protéger les sondes 23 et 24 du rayonnement émis par la paroi 32 qui est plus chaude. Au cas où ce rayonnement provoquerait une élévation de température du tube 22, on peut prévoir à l'intérieur de celui-ci un écran anti-rayonnement comportant une surface réfléchissante en regard de la paroi interne du tube.

On évite naturellement que la température dans l'enceinte 15 ne s'élève au-delà de la température maximale de fonctionnement du psychromètre (température d'ébullition de l'eau).

Une cartouche chauffante 39 alimentée par un câble 40 est disposée dans un élargissement terminal du conduit 13, à proximité de l'orifice 19 mais en amont des capteurs 25 et 26. La cartouche 39 est prévue pour réchauffer à peu près uniformément l'air qui la traverse, en vue d'éviter des phénomènes de condensation.

En effet, l'air chaud, lorsqu'il passe de la conduite 13 à la chambre 17, subit une certaine baisse de pression et de température qui aurait été susceptible de provoquer de la condensation si l'air n'était pas réchauffé au préalable, le réchauffage par la cartouche 30 étant également utile à cause des pertes de charge et des pertes thermiques se produisant entre la prise d'air 12 et la partie terminale de la conduite 13.

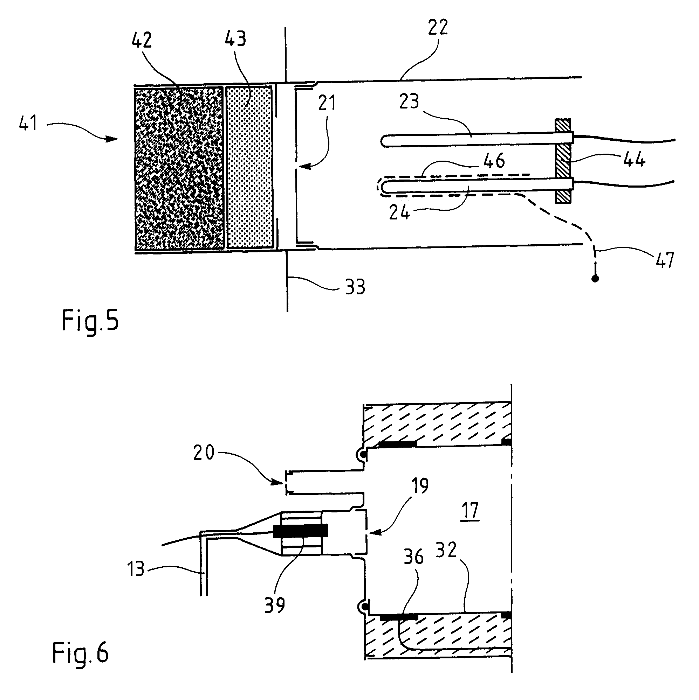

Un filtre 41 est prévu entre les chambres 17 et 18, et plus précisément dans la chambre 17 en amont de l'orifice 21. Le filtre 41 comporte un premier tampon 42 qui est ici en laine de roche, et un deuxième tampon 43, qui est ici en bronze fritté (voir figures 4 et 5). Ce filtre sert à protéger le psychromètre des poussières pouvant se trouver dans l'air mélangé.

Les sondes 23 et 24 sont disposées dans le conduit 22 côte-à-côte et au même niveau grâce à un support 44, la sonde de température humide 24 étant surplombée par l'extrémité d'un tube 45 d'alimentation en eau de mouillage pour l'habillage 46 en forme de doigt de gant dont est revêtue la sonde 24. Une mèche 47 rattachée à l'habillage 46 est prévue pour éliminer en dehors du conduit 22 un éventuel excès d'eau, tant que cet excès demeure modéré.

Le tube 45 est en matériau thermiquement conducteur, ici en cuivre, et il comporte dans la chambre 18 un certain nombre d'enroulements 48, dont seulement trois sont montrés sur la figure 3, afin qu'il ait dans cette chambre une longueur suffisante pour que l'eau de mouillage y séjourne suffisamment longtemps pour que les gouttes déposées sur l'habillage 46 soient à la température de la chambre 18. On évite ainsi d'avoir à contrôler et à réguler la température de l'eau de mouillage.

Dans l'exemple illustré, le débit de l'eau de mouillage (quelques gouttes par minute) a été déterminé par l'expérience, mais en variante on peut réguler ce débit en surveillant les signaux issus de la sonde de température humide pour détecter toute brusque variation signifiant que l'habillage 46 est en train de se déssécher, et agir en conséquence sur un organe de contrôle du débit dans le tube 45 pour réhumidifier l'habillage 46.

La source de succion 16 est formée par un tube éjecteur 49 alimenté en air comprimé par le tube 50 de sorte que de l'air est aspiré par le tube 51 qui relie le tube 49 à la chambre 18. On observera que le tube 51 débouche à la partie la plus basse de la chambre 18 de sorte qu'un éventuel excès d'eau qui y aurait été amené par la mèche 47 serait également aspiré.

Ainsi qu'indiqué précédemment, la pression de l'air comprimé dans le tube 50 est maintenue constante grâce à un régulateur de pression non représenté, qui est ici directement celui de la source d'air comprimé.

D'une façon générale, la valeur de cette pression ainsi que le dimensionnement du mélangeur 14 (volume des chambres 17 et 18, calibrage des orifices 19 à 21 et section du conduit 22) sont choisis pour avoir une bonne qualité de mélange dans la chambre 17, et une vitesse de l'air mélangé dans le conduit 22 qui conviennent à l'utilisation d'un psychromètre, c'est-à-dire une vitesse de l'ordre de 2 m/s.

Le débit global à l'intérieur de l'enceinte 15 est ainsi relativement faible par rapport à la section des chambres 17 et 18, on notera d'ailleurs à cet égard que l'on a intérêt à avoir un débit faible pour maximiser la durée de vie du filtre 41.

On peut dans ces conditions considérer que la pression dans la chambre 17 reste constante et homogène, de sorte qu'il est inutile de prévoir un capteur pour la mesurer en permanence.

La localisation des orifices 19, 20 et 21, respectivement au centre de la paroi 34, dans la paroi 32 et au centre de la paroi 33 a été également choisie en fonction des exigences précitées, mais d'autres localisations peuvent convenir, et notamment celle montrée sur la figure 6 où l'orifice 20 est prévu à l'extrémité d'un tube qui débouche dans la paroi 34.

Dans une variante non représentée, le calculateur 11 est supprimé, sa fonction étant directement remplie par l'automate 8, celui-ci servant également à réguler le débit de l'eau de mouillage dans le tube 45 suivant la méthode précitée où l'on surveille le signal de la sonde de température 24, l'automate 8 pilotant également les colliers chauffants 36 et la cartouche 39 en fonction de la température dans l'enceinte 15 donnée par la sonde de température sèche 23.

Dans une autre variante non illustré, utilisable lorsque les circonstances le permettent, on estime simplement la température, la pression et l'humidité de l'air frais, voire la température et la pression de l'air chaud, plutôt que de les mesurer avec les capteurs et sondes 25 à 29.

On notera enfin qu'en fonction de la précision recherchée, on peut remplacer les psychromètres par des capteurs d'humidité d'un autre type ; qu'on peut utiliser le dispositif décrit pour mesurer l'humidité de toute sorte d'air autre que celui d'une étuve de séchage ; et qu'en cas de variation de température importante de l'air à mesurer, on peut obturer l'orifice d'air frais au cours des périodes où les caractéristiques de l'air chaud permettent de mesurer directement son humidité par le capteur prévu dans la chambre de mesure.

Bien entendu, l'invention ne se limite aux exemples décrits et représentés.

## Revendications

1.  Procédé pour mesurer l'humidité d'un air chaud, caractérisé en ce qu'on dilue dans des conditions connues ledit air chaud avec de l'air frais dont l'humidité est connue afin d'obtenir un air mélangé dont la température est plus basse, on mesure l'humidité dudit air mélangé, et on déduit l'humidité de l'air chaud à partir des conditions connues de dilution, de l'humidité connue de l'air frais et de l'humidité mesurée de l'air mélangé.

2.  Procédé selon la revendication 1, caractérisé en ce que pour obtenir ledit air mélangé, on utilise une source de succion (16), une chambre de mesure (18) qui ne s'ouvre que sur la source de succion (16) et sur une chambre de mélange (17), et ladite chambre de mélange (17) qui ne s'ouvre que sur la chambre de mesure (18), sur l'air chaud et sur l'air frais, la dilution s'effectuant en régime continu dans la chambre de mélange (17) tandis que la chambre de mesure (18) est traversée par un flux régulier d'air mélangé qui provient de la chambre de mélange (17) et se dirige vers la source de succion (16).

3.  Procédé selon la revendication 2, caractérisé en ce que pour connaître les conditions de dilution :
    - on prédétermine le calibrage des orifices (19, 20) à travers lesquels la chambre de mélange (17) s'ouvre respectivement sur l'air chaud et sur l'air frais ;
    - on maintient à une valeur constante prédéterminée la pression de la source de succion (16) ; et
    - on mesure ou on estime la pression et la température de l'air frais et de l'air chaud en amont de leur orifice calibré respectif (19, 20).

4.  Procédé selon l'une quelconque des revendications 2 ou 3, caractérisé en ce que pour éviter des phénomènes de condensation, on réchauffe ledit air chaud en amont de la chambre de mélange (17), et on chauffe les parois (32, 34) des chambres de mélange et de mesure (17, 18).

5.  Procédé selon l'une quelconque des revendications 2 à 4, caractérisé en ce que pour mesurer l'humidité de l'air mélangé, on utilise un psychromètre (23, 24) disposé dans ladite chambre de mesure (18).

6.  Dispositif pour mesurer l'humidité d'un air chaud, convenant à la mise en oeuvre du procédé selon l'une quelconque des revendications 1 à 5, caractérisé en ce qu'il comporte :
    - un mélangeur (14) pour diluer dans des conditions connues ledit air chaud avec de l'air frais dont l'humidité est connue, afin d'obtenir un air mélangé dont la température est plus basse ; et
    - un capteur (23, 24) pour mesurer l'humidité dudit air mélangé, destiné à être relié à un calculateur (11) adapté à déduire l'humidité de l'air chaud à partir des informations fournies par ledit capteur (23, 24), de l'humidité connue de l'air frais, et des conditions connues de dilution.

7.  Dispositif selon la revendication 6, caractérisé en ce que ledit mélangeur comporte une enceinte (15) destinée à communiquer avec la source d'air chaud (1), avec la source d'air frais et avec une source de succion (16) ; ladite enceinte (15) étant divisée en une chambre de mélange (17) et une chambre de mesure (18) ; ladite chambre de mélange (17) ne communiquant qu'avec la source d'air chaud (1), la source d'air frais et la chambre de mesure (18) ; ladite chambre de mesure (18) ne communiquant qu'avec la chambre de mélange (17) et la source de succion (16) de sorte que la dilution s'effectue en régime continu dans la chambre de mélange (17) tandis que la chambre de mesure (18) est traversée par un flux régulier d'air mélangé qui provient de la chambre de mélange (17) et se dirige vers la source de succion (16) ; ledit capteur (23, 24) pour mesurer l'humidité de l'air mélangé étant disposé dans ladite chambre de mesure (18).

8.  Dispositif selon la revendication 7, caractérisé en ce que ledit mélangeur (14) comporte un premier orifice à calibrage prédéterminé (19) entre ladite chambre de mélange (17) et la source d'air chaud (1), et un deuxième orifice à calibrage prédéterminé (20) entre la chambre de mélange (17) et la source d'air frais.

9.  Dispositif selon la revendication 8, caractérisé en ce qu'il comporte en outre :
    - des capteurs (25, 26) pour mesurer la pression et la température de l'air chaud en amont dudit premier orifice à calibrage prédéterminé (19) ; et
    - des capteurs (27, 28, 29) pour mesurer l'humidité, la température et la pression de l'air frais en amont dudit deuxième orifice à calibrage prédéterminé (20) ;

    la pression de la source de succion (16) étant prévue pour rester constante.

**10.** Dispositif selon l'une quelconque des revendications 8 ou 9, caractérisé en ce que ledit mélangeur (14) comporte des moyens pour réchauffer ledit air chaud entre la source d'air chaud (1) et l'amont dudit premier orifice à calibre prédéterminé (19).

**11.** Dispositif selon l'une quelconque des revendications 6 à 10, caractérisé en ce que ledit mélangeur (14) comporte des moyens (36) pour chauffer les parois des chambres de mélange et de mesure (17, 18).

**12.** Dispositif selon l'une quelconque des revendications 7 à 11, caractérisé en ce que ledit capteur pour mesurer l'humidité de l'air mélangé est un psychromètre (23, 24), et ledit mélangeur (14) comporte dans ladite chambre de mesure (18) un conduit (22) de section prédéterminée dans lequel est disposé le psychromètre, ledit conduit (22) communiquant avec ladite chambre de mélange (17) à travers un troisième orifice à calibrage prédéterminé (21).

**13.** Dispositif selon la revendication 12, caractérisé en ce que le psychromètre comporte un tube (45) d'alimentation en eau qui a dans la chambre de mesure (18) une longueur adaptée à amener à la température de la chambre de mesure l'eau qui se dépose sur la sonde (24) de température humide.

**14.** Dispositif selon l'une quelconque des revendications 7 à 13, caractérisé en ce que ledit mélangeur (14) comporte un filtre entre la chambre de mélange (17) et la chambre de mesure (18).

**15.** Installation de séchage à air chaud, caractérisée en ce qu'elle comporte un dispositif selon l'une quelconque des revendications 6 à 14 pour mesurer l'humidité de l'air chaud de séchage.

**16.** Installation selon la revendication 15, caractérisée en ce qu'elle comporte des moyens (13, 13', 31) pour que ledit dispositif puisse mesurer l'humidité de l'air chaud de séchage respectivement en amont et en aval du chargement à sécher (2).

**17.** Installation selon l'une quelconque des revendications 15 ou 16, caractérisée en ce qu'elle comporte un automate (8) qui pilote au moins un de ses organes en fonction des informations d'humidité issues dudit dispositif pour mesurer l'humidité de l'air chaud de séchage.

**18.** Installation selon la revendication 17, caractérisée en ce que ledit automate (8) pilote en fonction des informations fournies par le dispositif de mesure d'humidité au moins l'un des organes suivants :

- organe d'humidification de l'air de séchage (7A, 7B) ;
- organe d'introduction d'air neuf (6A, 6B) ;
- organe d'extraction d'air de séchage (6A, 6B) ;
- organe de circulation d'air (4) ; et
- organe de chauffage (3).

Fig.1

Fig.2

Fig.3

Fig.4

Fig.5

Fig.6

EP 0 633 461 A2